# EUROPÄISCHE PATENTSCHRIFT

(11) **EP 1 319 372 B1**
(45) Veröffentlichungstag und Bekanntmachung des Hinweises auf die Patenterteilung: **25.06.2003**
(21) Anmeldenummer: 02010059.0
(22) Anmeldetag: 06.05.2002
(51) Int. Cl.: A61B 17/28

(54) **Vorrichtung zum Halten von Trokarhülsen**
Device for holding a trocar sleeve
Dispositif pour tenir un manchon de trocart

(43) Veröffentlichungstag der Anmeldung: 18.06.2003
(73) Patentinhaber: Karl Storz GmbH & Co. KG, 78532 Tuttlingen (DE)
(72) Erfinder: Mosnier, Henry, 92500 Rueil Malmaison (FR); Lafond, Christophe, 60200 Compiègne (FR)
(74) Vertreter: Weller, Wolfgang, Dr.

(56) Entgegenhaltungen:
- DE-A- 4 115 548
- US-A- 5 626 597
- US-A- 6 039 725

## Beschreibung

Die vorliegende Erfindung betrifft eine Vorrichtung zum Halten einer in einem Körper eines Patienten steckenden Trokarhülse in Position, mit Klemmbacken, welche in Schließstellung eine Öffnung umgrenzen und welche um die Außenseite einer Trokarhülse legbar sind und mit zumindest einer Öffnung, über die die Zange am Patienten fixierbar ist.

Eine derartige Vorrichtung ist in Form einer von der Anmelderin unter der Nummer 27105 AG vertriebenen Fixierscheibe bekannt. Derartige Haltevorrichtungen werden dazu verwendet, um Trokarhülsen oder ähnliche chirurgische Instrumente in einer bestimmten Position zu halten.

US 6,039,725 offenbart eine Vorrichtung zum halten von Trokarhülsen entsprechend des Oberbegriffs von Anspruch 1.

Nachteilig bei den bekannten Trokarfixierscheiben ist jedoch, daß sie aufgrund ihrer relativ großen ausladenen Fläche und deren Starrheit insbesondere bei unebenen Körperflächen nicht angewendet werden können. Dies ist beispielsweise bei Operationen im Hals- oder Nackenbereich der Fall, wo die Körperoberfläche des Patienten keine Möglichkeit bietet, eine Haltevorrichtung in Form einer ebenen, flachen Platte oder Scheibe zu befestigen.

Weiterhin nachteilig ist, daß durch die Scheiben ein relativ großer Bereich auf dem Patientenkörper abgedeckt wird, wodurch dieser für den behandelnden Arzt weder einsehbar noch für andere Instrumente zugänglich ist. Bei minimal-invasiven Eingriffen werden üblicherweise mehrere Trokare gesetzt, z.B. einer zur Beobachtung der Operationsstelle mit einem Endoskop und ein zweiter für das eigentliche Operationsinstrument.

Aufgrund der durch die Scheibe relativ groß abgedeckten Fläche ist es außerdem nicht möglich, in der Nähe der eingeführten Trokarhülse weitere Operationsinstrumente in den Körper des Patienten einzuführen oder auf dem Körper zu fixieren.

Es sind darüber hinaus automatisierte roboterähnliche Trokar- und Instrumenten-Positionierungssysteme bekannt. Derartige Vorrichtungen weisen mehrgliedrige Gelenkarme auf, deren einzelne Gliederelemente unabhängig voneinander in verschiedenen Achsen ausrichtbar sind. Damit kann das zu fixierende Instrument in einer beliebigen Position gehalten werden.

Derartige Vorrichtungen sind jedoch hochkompliziert und sowohl in der Herstellung als auch in der Handhabung sehr aufwendig und darüber hinaus äußerst kostspielig. Weiterhin benötigen derartige Haltesysteme äußerst viel Platz im Operationsraum und behindern außerdem durch ihre Anbringung neben dem Operationstisch und über dem Patienten den operierenden Arzt. Nachteilig an diesen Systemen ist weiterhin, daß die Trokare hier lediglich relativ zu einer Operations-Einrichtung fixiert werden können.

Daher ist es Aufgabe der vorliegenden Erfindung, eine Vorrichtung zum Halten einer Trokarhülse zu schaffen, die einfach bedienbar ist, die wenig raumgreifend ist und die auch auf unebenen Flächen sicher fixiert werden kann.

Diese Aufgabe wird dadurch gelöst, daß die Vorrichtung als Zange mit zwei Zangenschenkeln ausgebildet ist, die über ein Scharnier miteinander verbunden sind und die an ihren distalen Enden jeweils zumindest eine Klemmbacke tragen, wobei zwischen den Zangenschenkeln eine Feder angeordnet ist, welche über ihre langgezogenen Enden an den Zangenschenkeln abgestützt ist und welche die Klemmbacken in die Schließstellung drückt.

Die erfindungsgemäße Vorrichtung bietet den Vorteil, daß sie aufgrund ihrer Ausgestaltung als Zange auch bei unebenen Körperoberflächen zum Halten einer Trokarhülse verwendet werden kann, da die Schenkel in eine jeweils günstige Position verschwenkt werden können und seitlich vom zu haltenden Trokar wegstehen.

Weiterhin bietet die erfindungsgemäße Vorrichtung den Vorteil, daß in unmittelbarer Nähe zu einer durch eine derartige Vorrichtung fixierten Trokarhülse weitere Operationsinstrumente und/oder Trokare in den Körper eines Patienten eingeführt und ebenfalls fixiert werden können. Dies ist bspw. notwendig, wenn über eine fixierte Trokarhülse chirurgische Instrumente eingeführt werden, mit denen innere Gewebe oder Organe behandelt werden können, wobei diese Schritte gleichzeitig über ein Endoskop in einer unmittelbar benachbart gesetzten Trokarhülse beobachtet werden sollen. Die die fixierten Trokarhülsen umgebende Körperoberflächen bleiben für den behandelnden Arzt dennoch einsehbar.

Die Klemmbacken der erfindungsgemäßen Vorrichtung, die durch eine Feder in Schließstellung gehalten werden, können durch Zusammendrücken der Zangenschenkel gegen den Druck der Feder geöffnet werden. Die geöffneten Klemmbacken können dann um eine Trokarhülse gelegt werden. Durch Loslassen der Zangenschenkel werden aufgrund der Kraft der Feder die Klemmbacken an die Außenseite der Trokarhülse gedrückt, wodurch die Zange unverrückbar an der Trokarhülse festsitzt. Auf diese Weise wird sichergestellt, daß die auf diese Weise in axialer Richtung fixierte Trokarhülse nicht mehr weiter eindringen kann. Mögliche innere Verletzungen, die durch ein zu tiefes Eindringen einer nicht fixierten Trokarhülse hervorgerufen werden können, werden dadurch vermieden. Ein Operateur kann die Zange, falls die Zangenschenkel eine Manipulation stören, einfach ergreifen, den Anpreßdruck etwas lösen und die Vorrichtung um die Trokarachse verschwenken.

In einer bevorzugten Ausführungsform ist die zumindest eine Öffnung in einer schwenkbaren Halterung vorhanden, die an der erfindungsgemäßen Vorrichtung angebracht ist.

Die Halterung kann dabei an den Zangenschenkeln oder über das die Zangenschenkel verbindende Scharnier schwenkbar angebracht sein. Die Halterung ist dabei beispielsweise in Form einer länglichen flachen Lasche ausgebildet, die die zumindest eine Öffnung aufweist.

Die Halterung kann nur eine oder auch mehrere Öffnungen aufweisen, über die die Vorrichtung am Patientenkörper fixiert werden kann. Weiterhin können mehrere Halterungen mit zumindest einer Öffnung vorgesehen sein.

Dabei ist vorteilhaft, wenn die Halterung in Form einer länglichen flachen Lasche ausgebildet ist und über das Scharnier angebracht ist. Dadurch können die Zangenschenkel auch dann zusammengedrückt werden, wenn die Zange über die in der Halterung vorliegende Öffnung durch Annähen am Patientenkörper fixiert ist.

Diese Maßnahme hat weiterhin den Vorteil, daß die Vorrichtung insgesamt im fixierten Zustand um das Scharnier als Drehpunkt verschwenkt werden kann.

Die schwenkbare Halterung kann in einer bestimmten Schwenkstellung relativ zu einer der Klemmbacken oder relativ zu einer Mittelebene zwischen den Klemmbacken feststellbar ausgebildet sein.

In einer bevorzugten Ausführungsform weist die Vorrichtung zumindest zwei Öffnungen seitlich am distalen Endbereich der Zangenschenkel nahe der Klemmbacken auf.

Über diese Öffnungen kann die Vorrichtung, deren Klemmbacken um die Trokarhülse gelegt sind, am Patientenkörper bspw. durch Nähen fixiert werden und dann zusätzlich ein ungewolltes Abziehen der Trokarhülse verhindern. Hat sich der Arzt auf die Stellung der Trokarhülse relativ zum Patienten festgelegt, kann er die Klemmbacken der Vorrichtung um die Trokarhülse legen, diese dadurch festklemmen und anschließend die Vorrichtung über die Öffnungen am Patienten festnähen, wodurch die Trokarhülse auch in ihrer relativen Lage zum Patientenkörper axial in beiden Richtungen fixiert ist. Die Form der Öffnungen kann dabei variieren und einen runden, eckigen oder langgezogenen Umfang aufweisen.

Die Vorrichtung kann auch durch Kleben am Patientenkörper befestigt werden.

Die Vorrichtung kann außerdem mehr als zwei Öffnungen aufweisen, über die die Vorrichtung noch stabiler am Patientenkörper fixiert werden kann.

Die erfindungsgemäße Vorrichtung bietet so insbesondere den Vorteil, daß v.a. bei Operationen im Hals/-Nackenbereich die Trokarhülsen stabil gehalten und fixiert werden können, so daß unerwünschte Relativbewegungen der zu handhabenden Instrumente in Bezug zu dem Patienten und damit verbundene Verletzungsrisiken des Rückenmarks und/oder der Wirbelsäule vermieden werden.

Die erfindungsgemäße Vorrichtung bietet darüber hinaus gegenüber Fixierhilfen mit Schraub- oder Spannelementen den Vorteil, daß durch sie die Trokarhülse auf die oben beschriebene Weise mit einem einzigen, einfachen Handgriff fixiert werden kann. Die Vorrichtung kann mit einer Hand betätigt und durch einfaches Drücken und Entlasten geöffnet und geschlossen werden. Dadurch können Operationsschritte schneller und einfacher durchgeführt werden.

Die Länge der Zangenschenkel kann dem jeweiligen Einsatzgebiet angepaßt sein, und insbesondere bei Operationen am Nacken kurz sein. Dadurch wird gewährleistet, daß die Zangenschenkel den behandelnden Arzt bei anschließenden weiteren chirurgischen Eingriffen nicht behindern.

Bei einer weiteren bevorzugten Ausführungsform sind die langgezogenen Enden der Feder über zwei von den Zangenschenkeln abnehmbare Bolzen abgestützt.

Durch diese Maßnahme wird erreicht, daß die Feder stabil zwischen den Zangenschenkeln befestigt liegt. Zum Reinigen werden die Bolzen abgenommen und die Feder vom Zangenkörper getrennt.

Bei einer weiteren Ausgestaltung der Erfindung sind die Bolzen am proximalen Teil der Zangenschenkel angeordnet.

Diese Maßnahme hat den Vorteil, daß sich die Schenkel der Feder längs der Zangenschenkel erstrecken und keine raumgreifende und sichtbehindernde Bauteile darstellen.

Die Bolzen können insgesamt massiv oder aber, zur Verminderung des Gewichts, hohl sein und können bspw. zylinder-, kegelförmig oder eckig, symmetrisch oder asymmetrisch ausgebildet sein.

In einer weiteren Ausführungsform weisen die Zangenschenkel jeweils zwei Klemmbackenpaare auf, wobei jeweils zwei Klemmbacken in Schließstellung je eine Öffnung im Abstand voneinander umgrenzen.

Diese Maßnahme hat den Vorteil, daß mit der Vorrichtung gleichzeitig zwei Trokare gehalten werden können. Dadurch muß nur eine Vorrichtung verwendet werden, wodurch insgesamt weniger Instrumente bei einem Eingriff gehandhabt werden müssen.

In einer Weiterbildung der Ausführungsform ist der Abstand der durch die Klemmbacken gebildeten Öffnungen verstellbar.

Diese Maßnahme hat den Vorteil, daß bei einem Einsatz von bspw. zwei Trokaren diese in einem flexiblen Abstand zueinander in einen Patientenkörper eingeführt werden können. Die Klemmbacken können dabei auf den hierfür schienenartig ausgebildeten distalen Enden der Zangenschenkel verschoben werden und dem Abstand der Trokare voneinander angepaßt werden. Über bspw. seitlich durch die Klemmbacken einzuführende Einstellschrauben können die Klemmbacken auf den Zangenschenkeln fixiert werden.

Mit der Vorrichtung können dann beide Trokare über die durch die Klemmbacken gebildeten Öffnungen fixiert werden. Anschließend wird die Vorrichtung am Patientenkörper beispielsweise durch Nähen befestigt.

In einer weiteren bevorzugten Ausführungsform weisen die Klemmbacken eine Fläche auf, die im Querschnitt senkrecht zur Trokarachse kreisbogenförmig ist. Hierbei ist bevorzugt, wenn die Fläche zur durch die Klemmbacken gebildeten Öffnung hin konkav ausgebildet ist.

Dadurch wird erreicht, daß die Klemmfläche der Klemmbacken den Trokarhülsen angepaßt ist. Trokarhülsen weisen meist eine zylindrische Form auf, bzw. sind an der Stelle, an die die Zange angelegt werden soll, zylindrisch ausgebildet. Durch die kreisbogenförmige Ausgestaltung der Klemmbacken wird der auf die Außenwand der Trokarhülse von der Feder der Zange ausgeübte Druck gleichmäßig auf den Umfang der zylindrische Fläche der Trokarhülse verteilt.

Die Form der durch die Klemmbacken gebildeten Öffnung kann aber auch bspw. insgesamt eckig und symmetrisch oder asymmetrisch geformt sein, insbesondere an die Außenflächenform der Trokarhülse angepaßt sein. Die Klemmbacken können weiterhin auf ihrer der Öffnung zugewandten Seite bspw. eine geriffelte Oberfläche aufweisen, durch welche die Trokarhülse noch besser gefaßt werden kann.

In einer bevorzugten Ausführungsform ist die Öffnung der Vorrichtung von ca. 3 bis 5 mm groß. Auf diese Weise können Trokarhülsen mit einem geringeren Durchmesser stabil gehalten werden.

In einer anderen Ausführungsform bilden die Klemmbacken in der Schließstellung eine Öffnung von 5 bis 10 mm. Dadurch können auch Trokarhülsen mit einem größeren Durchmesser zum Halten in der Öffnung der Vorrichtung angebracht sein. Dies ist beispielsweise für Vorrichtungen wünschenswert, die bei laparoskopischen Eingriffen eingesetzt werden.

Dabei ist bevorzugt, wenn die erfindungsgemäße Vorrichtung aus einem Material besteht, ausgewählt aus Metall, insbesondere Leichtmetall, und Kunststoff.

Weiterhin ist bevorzugt, wenn das Material Aluminium ist.

Diese Maßnahmen haben den Vorteil, daß die Vorrichtung sehr leicht ist. Das Eigengewicht der Zange beschwert die mit ihr umklammerte Trokarhülse nicht erheblich, so daß keine Gefahr besteht, daß die Trokarhülse durch das Gewicht der Zange aus seiner Position im Patientenkörper herausgezogen wird. Durch das leichte Gewicht ist die Vorrichtung darüber hinaus besonders einfach und mit nur einer Hand zu bedienen.

Weiterhin ist die Vorrichtung insbesondere wegen ihrer kleinen Ausführung und wegen ihres leichten Gewichts vergleichsweise kostengünstig herzustellen.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen dargestellt und werden in der nachfolgenden Beschreibung näher erläutert. Es zeigen:
- Fig. 1: eine schematische, perspektivische Darstellung einer Ausführungsform der erfindungsgemäßen Vorrichtung;
- Fig. 2: eine schematische Darstellung einer anderen Ausführungsform der erfindungsgemäßen Vorrichtung in Aufsicht;
- Fig. 3: eine schematische Darstellung einer weiteren Ausführungsform der erfindungsgemäßen Vorrichtung in Aufsicht; und
- Fig. 4: eine schematische, teilweise vergrößerte Darstellung einer Verwendung der erfindungsgemäßen Vorrichtung aus Fig. 1, in Position auf einem Patientenkörper.

Die in den Figuren dargestellten Vorrichtungen zeigen mit 10 die Zange insgesamt, wobei mit 12 und 13 die beiden Zangenschenkel bezeichnet sind. Die beiden Zangenschenkel 12 und 13 sind über ein Scharnier 14 im Kreuzungsbereich miteinander verbunden. Eine zentrale spiralförmige Feder 16 hält die Zangenschenkel 12 und 13 in der Schließendstellung auseinander gedrückt.

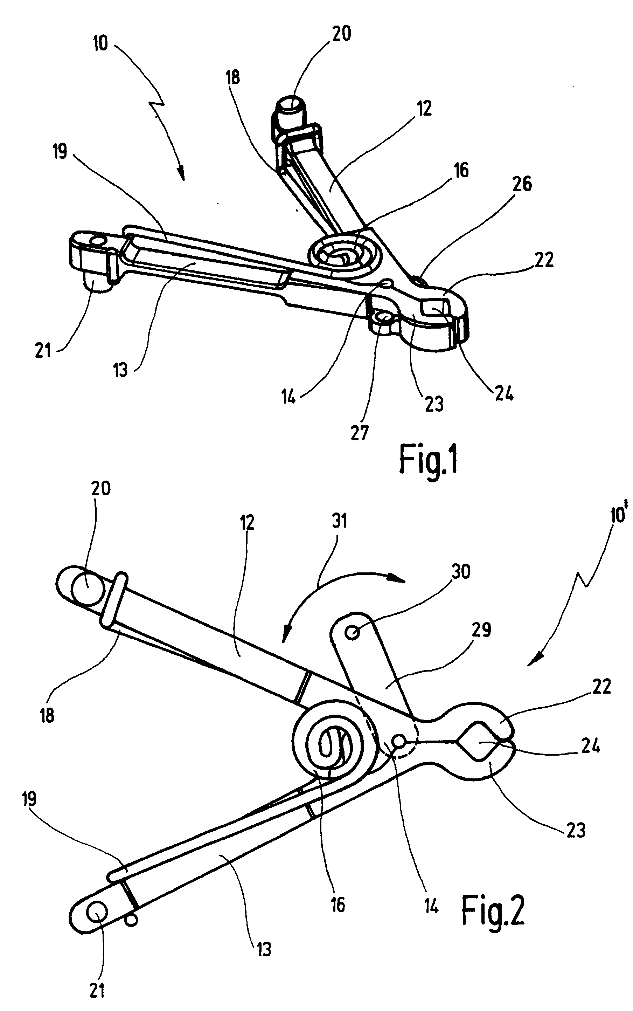

Die langgezogenen Enden 18 und 19 der spiralförmigen Feder 16 sind jeweils über Bolzen 20 und 21 an den Zangenschenkeln 12 und 13 abgestützt, wobei die Bolzen 20 und 21 jeweils an den Zangenschenkeln 12 und 13 angebracht sind. Wie in Fig. 2 gezeigt, ist der Abschnitt 19 der Feder 16 oberhalb des Zangenschenkels 13 und an diesem entlang gelegen, am proximalen Ende der Zange abgewinkelt um den Zangenschenkel 13 geführt und an dem dort angebrachten Bolzen 21 abgestützt. Aufgrund der Ausführungsform der Feder 16 ist der Abschnitt 18 unterhalb des Zangenschenkels 12 und an diesem entlang gelegen, am proximalen Ende abgewinkelt um den Zangenschenkel 12 geführt und an dem dort angebrachten Bolzen 20 abgestützt.

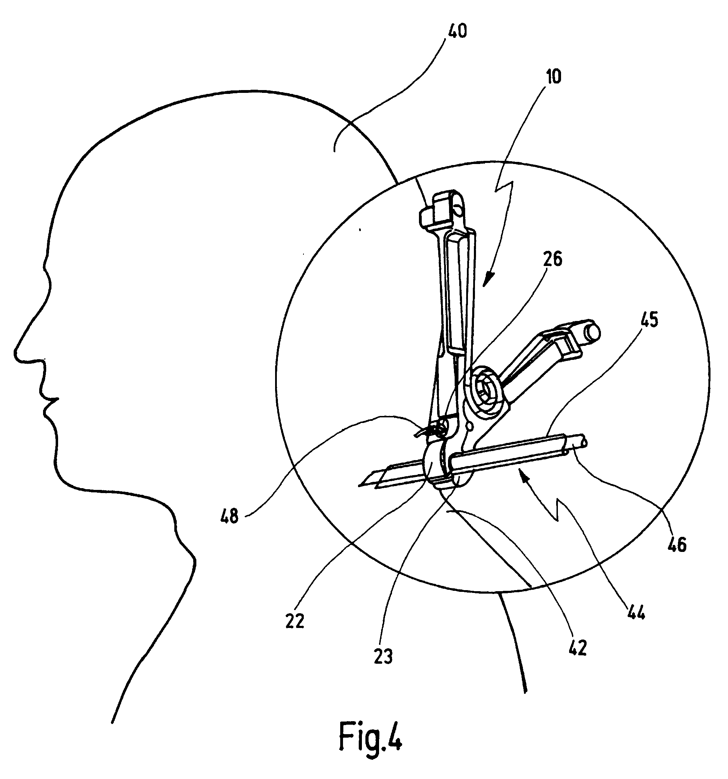

Die zwei Zangenschenkel 12 und 13 weisen an ihrem distalen Ende jeweils eine Klemmbacke 22 und 23 auf, durch welche in der geschlossenen Endstellung eine Öffnung 24 gebildet wird, in welcher eine Trokarhülse 45 gehalten werden kann, wie es in Fig. 4 gezeigt ist.

In Fig. 1 weist die Zange 10 an den Seiten der Zangenschenkel 12 und 13 unterhalb der Klemmbacken 22 und 23 rechts und links zwei augenartige Öffnungen 26 und 27 auf, über welche die Zange 10 an eine Körperoberfläche eines Patienten 40 mittels eines chirurgischen Fadens 48 fixiert werden kann.

In den Fig. 2 ist bei der Zange 10' eine Halterung 29 vorgesehen, die laschenförmig ausgebildet ist und die eine augenförmige Öffnung 30 aufweist. Die Halterung 29 ist dabei schwenkbar über das Scharnier 14 an der Zange 10' angebracht, was durch den Pfeil 31 angedeutet ist. Über die in der Halterung 29 vorgesehene augenförmige Öffnung 30 kann die Zange 10' durch Annähen am Patientenkörper 40 fixiert werden.

Da die Halterung 29 schwenkbar angebracht ist, ist es weiterhin möglich, die Zangenschenkel 12 und 13 auch dann zusammenzudrükken und die dadurch geöffneten Klemmbacken 22 und 23 um einen Trokar zu legen, wenn die Zange 10' insgesamt bereits über die Öffnung 30 der Halterung 29 am Patientenkörper fixiert ist.

Bei einem operativen Eingriff greift der behandelnde Arzt die beiden Zangenschenkel 12 und 13 und drückt diese gegen die Kraft der Feder 16 zusammen. Dadurch öffnen sich die Klemmbakken 23 und 24, wodurch es möglich ist, diese um eine Trokarhülse zu legen.

Die Zangenschenkel 12 und 13 können dabei, wie in Fig. 2 dargestellt ist, bspw. in dem Abschnitt, der zwischen dem die Bolzen 20 und 21 tragenden Ende und dem Kreuzungsbereich liegt, mit einem dünneren Durchmesser ausgebildet sein. Dadurch kann die Zange sowohl besser gegriffen als auch insgesamt das Gewicht der Zange weiter verringert werden.

Durch Loslassen der Zangenschenkel 12 und 13 werden die Klemmbacken 22 und 23 durch die Feder 16 wieder in Schließstellung gedrückt, wodurch die Klemmbacken 22 und 23 nun die Trokarhülse fest umschließen.

Die Größe der durch die beiden Klemmbacken 22 und 23 gebildeten Öffnung 24 hängt von der zu verwendenden Trokarhülse ab und beträgt beispielsweise 3 bis 5 mm, mit kurzen Zangenschenkeln insbesondere für Eingriffe im Nackenbereich. Die Ausführungsgröße der Öffnung 24 bei anderen Ausführungsformen kann beispielsweise bei 5 mm bis 10 mm liegen, Trokare mit einer derartigen Öffnung werden beispielsweise für alle laparoskopischen Eingriffe eingesetzt.

Die Form der durch die Klemmbacken 22 und 23 gebildeten Öffnung 24 kann variieren und dem Umfang der zu haltenden Trokarhülse angepaßt sein. So kann die Öffnung bspw. rund oder eckig, symmetrisch oder asymmetrisch etc. sein. Die der Öffnung zugewandten Fläche der Klemmbacken 22 und 23 kann darüber hinaus Längsrillen und/oder Querrillen aufweisen, die einem noch besseren Griff dienen.

Die dadurch in der Zange 10 bzw. 10' gehaltene Trokarhülse kann sich somit in axialer Richtung nicht verschieben, wodurch eine erhebliche Verletzungsgefahr durch ein weiteres Eindringen der Trokarhülse in den Patienten vermieden wird.

Die um eine Trokarhülse gelegte Zange 10 bzw. 10' kann anschließend dadurch fixiert werden, daß sie über die Öffnungen 26, 27 oder 30 am Patientenkörper festgenäht wird. Dadurch wird vermieden, daß die Zange durch deren Eigengewicht von der Oberfläche des Patienten abrutscht. Durch ein vorzugsweise leichtes Gewicht der Zange wird außerdem eine Belastung des Patienten vermieden. Ein leichtes Gewicht wird durch Verwendung eines leichten Metalls, wie bspw. Aluminium, oder eines Kunststoffs gewährleistet.

Die Zange kann aber auch bspw. durch Kleben am Patientenkörper befestigt werden.

Die Öffnungen 26, 27 und 30 sind in den Fig. 1 bis 3 gezeigten Darstellungen rund, können jedoch auch eckig oder langgezogen ausgebildet sein und aus Abschnitten mit unterschiedlichen Durchmessern bestehen.

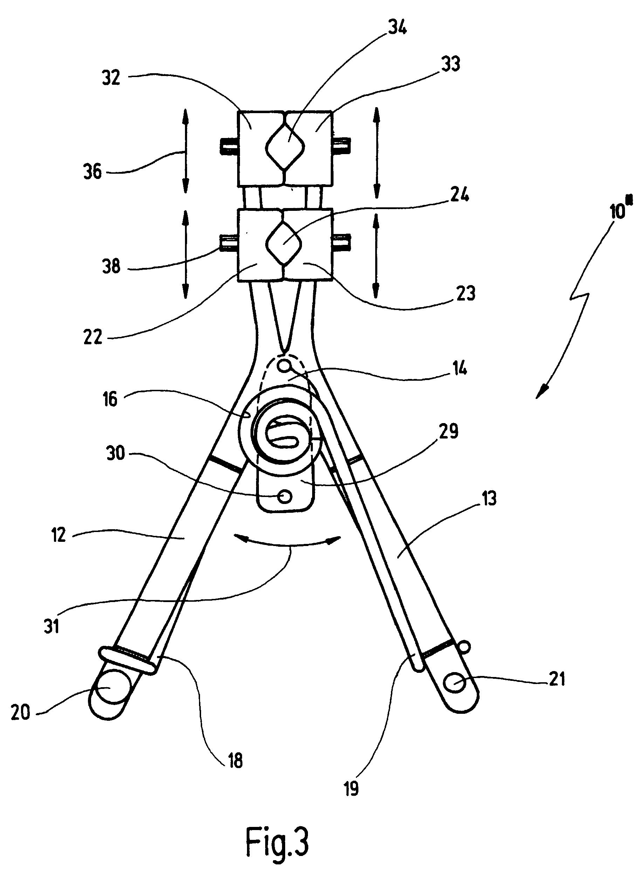

In Fig. 3 ist eine weitere Ausführungsform der erfindungsgemäßen Vorrichtung gezeigt.

Hierbei sind bei der Zange 10'' mit 32 und 33 zwei weitere Klemmbacken bezeichnet, die in Schließstellung eine zweite Öffnung 34 umschließen. Die Klemmbacken 22, 23, 32 und 33 sind auf den im distalen Bereich schienenartig ausgebildeten Zangenschenkeln 12 und 13 verschiebbar angebracht, was für die Klemmbacke 32 durch den Pfeil 36 beispielhaft angedeutet ist. Entsprechendes gilt für die Klemmbacken 23, 32 und 33.

Mit 38 ist eine Feststellschraube bezeichnet, die seitlich durch die Klemmbacke 22 geführt ist und über die die Klemmbacke 22 auf dem schienenartigen distalen Ende des Zangenschenkels 12 festgestellt werden kann. Ähnliche Feststellschrauben befinden sich entsprechend an den Klemmbacken 23, 32 und 33. Durch die verschiebbare Anordnung kann der Abstand zwischen den beiden durch die Klemmbacken 22, 23 und 32, 33 gebildeten Öffnungen 24 und 34 unterschiedlich festgelegt werden. Dadurch ist es möglich, daß beispielsweise ein erster Trokar mittels der erfindungsgemäßen Zange 10'' in der ersten Öffnung 24 gehalten wird und anschließend in der Umgebung des ersten eingeführten Trokars ein weiterer Trokar im Patientenkörper angebracht wird. Mittels des verschiebbaren Klemmbackenpaars 32, 33 kann dieser zweite Trokar in der Öffnung 34 der gleichen Zange 10'' fixiert werden.

Weiterhin besteht die Möglichkeit, nur das jeweils oberste Klemmbackenpaar verschiebbar anzuordnen und darüber hinaus noch weitere Klemmbacken an der Zange anzubringen.

Über die auf der schwenkbaren Halterung 29 vorgesehene Öffnung 30 kann die Vorrichtung durch Nähen am Patientenkörper fixiert werden.

In Fig. 4 ist ein beispielhafter Einsatz der erfindungsgemäßen Vorrichtung gezeigt. Die Vorrichtung und ein eingeführter Trokar 44 sind dabei in einem Ausschnitt vergrößert dargestellt.

Dabei wird an einem Patienten 40 in dessen Nackenbereich 42 ein operativer Eingriff vorgenommen. Hierzu ist ein Trokar 44 mit Trokarhülse 45 und Trokardorn 46 im Nackenbereich 42 des Patienten 40 eingeführt. Um die Trokarhülse 45 in axialer Richtung zu fixieren, sind die Klemmbacken 22 und 23 der Zange 10 um den Außenbereich der Trokarhülse 45 gelegt.

In einem nächsten Schritt wird die Zange 10 über die zwei Öffnungen 26 und 27 durch Annähen am Patienten 40 fixiert. Hierzu ist mit 48 ein Faden gezeigt, der durch die Öffnung 26 der Zange 10 in den Nackenbereich 42 des Patienten 40 geführt ist, wodurch die Zange 10 und dadurch auch der Trokar 44 fest im Nakkenbereich 42 fixiert ist.

Durch die geringe Größe der Zange 10 ist es weiterhin möglich, in unmittelbarer Nähe zu dem eingeführten und fixierten Trokar 44 weitere Operationsinstrumente einzuführen und auch mit weiteren Zangen zu fixieren, ohne daß der behandelnde Arzt durch die bereits angelegte Zange 10 behindert wäre.

## Patentansprüche

1. Vorrichtung zum Halten einer in einem Körper eines Patienten steckenden Trokarhülse in Position, mit Klemmbacken, welche in Schließstellung eine Öffnung umgrenzen und welche um die Außenseite einer Trokarhülse legbar sind und mit zumindest einer Öffnung, über die die Vorrichtung am Patienten fixierbar ist, **dadurch gekennzeichnet, daß** die Vorrichtung als Zange (10, 10', 10'') mit zwei Zangenschenkeln (12, 13) ausgebildet ist, die in ihrem Kreuzungsbereich über ein Scharnier (14) miteinander verbunden sind und die an ihren distalen Enden jeweils zumindest eine Klemmbacke (22, 23) tragen, wobei zwischen den Zangenschenkeln (12, 13) eine Feder (16) angeordnet ist, welche über ihre langgezogenen Enden (18, 19) an den Zangenschenkeln (12, 13) abgestützt ist und welche die Klemmbacken (22, 23) in die Schließstellung drückt.

2. Vorrichtung nach Anspruch 1, **dadurch gekennzeichnet, daß** die zumindest eine Öffnung (30) in einer schwenkbaren Halterung (29) vorhanden ist.

3. Vorrichtung nach Anspruch 1, **dadurch gekennzeichnet, daß** zwei Öffnungen (26, 27) seitlich am distalen Endbereich der Zangenschenkel (12, 13) nahe der Klemmbacken (22, 23) vorhanden sind, über die die Zange am Patienten fixierbar ist.

4. Vorrichtung nach einem der Ansprüche 1 bis 3, **dadurch gekennzeichnet, daß** die langgezogenen Enden (18, 19) der Feder (16) über zwei von den Zangenschenkeln (12, 13) abnehmbare Bolzen (20, 21) abgestützt sind.

5. Vorrichtung nach Anspruch 4, **dadurch gekennzeichnet, daß** die Bolzen (20, 21) im proximalen Teil der Zangenschenkel (12, 13) angebracht sind.

6. Vorrichtung nach einem der Ansprüche 1 bis 5, **dadurch gekennzeichnet, daß** die Zangenschenkel (12, 13) zwei Klemmbackenpaare aufweisen, wobei jeweils zwei Klemmbacken (22, 23) und (32, 33) in Schließstellung je eine Öffnung (24) und (34) im Abstand voneinander umgrenzen.

7. Vorrichtung nach Anspruch 6, **dadurch gekennzeichnet, daß** der Abstand der durch die Klemmbacken (22, 23 und 32, 33) gebildeten Öffnungen (24, 34) verstellbar ist.

8. Vorrichtung nach einem der Ansprüche 1 bis 7, **dadurch gekennzeichnet, daß** diese aus einem Material besteht, ausgewählt aus der Gruppe Metall, insbesondere Leichtmetall, und Kunststoff.

9. Vorrichtung nach Anspruch 8, **dadurch gekennzeichnet, daß** das Metall Aluminium ist.

## Claims

1. Device for holding a trocar sleeve in its position of engagement in the body of a patient, with clamping jaws which in the closed position define an opening and which can be placed about the outside of a trocar sleeve, and with at least one opening via which the device can be fixed on the patient, **characterized in that** the device is designed as a forceps (10, 10', 10'') with two forceps arms (12, 13) which at their area of intersection are connected to one another via a hinge (14) and which at their distal ends in each case have at least one clamping jaw (22, 23), a spring (16) being arranged between the forceps arms (12, 13), which spring (16) is supported on the forceps arms (12, 13) via its elongate ends (18, 19) and forces the clamping jaws (22, 23) into the closed position.

2. Device of claim 1, **characterized in that** the at least one opening (30) is present in a pivotable bracket (29).

3. Device of claim 1, **characterized in that** two openings (26, 27) are provided on the sides at the distal end area of the forceps arms (12, 13) near the clamping jaws (22, 23), via which two openings (26, 27) the forceps can be fixed on the patient.

4. Device of anyone of claims 1 through 3, **characterized in that** the elongate ends (18, 19) of the spring (16) are supported via two pins (20, 21) which are removable from the forceps arms (12, 13).

5. Device of claim 4, **characterized in that** the pins (20, 21) are arranged in the proximal part of the forceps arms (12, 13).

6. Device of anyone of claims 1 through 5, **characterized in that** the forceps arms (12, 13) have two pairs of clamping jaws, two clamping jaws (22, 23) and (32, 33) respectively defining, in the closed position, an opening (24) and (34) situated at a distance from one another.

7. Device of claim 6, **characterized in that** the distance between the openings (24, 34) formed by the clamping jaws (22, 23 and 32, 33) is adjustable.

8. Device of anyone of claims 1 through 7, **characterized in that** it is made of a material selected from the group comprising metal, in particular light metal, and plastic.

9. Device of claim 8, **characterized in that** the metal is aluminum.

## Revendications

1. Dispositif pour tenir en position une douille de trocart enfoncée dans le corps d'un patient, avec des mâchoires de blocage qui délimitent une ouverture dans la position de fermeture et qui peuvent être posées autour de la face extérieure d'une douille de trocart, et avec au moins une ouverture par l'intermédiaire de laquelle le dispositif peut être fixé sur le patient, **caractérisé en ce que** le dispositif est conformé en pince (10, 10', 10") avec deux branches de pince (12, 13), qui sont reliées par une charnière (14) dans leur zone de croisement et qui portent sur leurs extrémités distales chacune au moins une mâchoire de blocage (22, 23), un ressort (16) étant placé entre les branches de pince (12, 13), lequel est appuyé par ses extrémités allongées (18, 19) sur les branches de pince (12, 13) et presse les mâchoires de blocage (22, 23) dans la position de fermeture.

2. Dispositif selon la revendication 1, **caractérisé en ce que** l'ouverture (30) au nombre d'au moins une est prévue dans un support pivotant (29).

3. Dispositif selon la revendication 1, **caractérisé en ce que** deux ouvertures (26, 27) sont prévues latéralement sur la zone d'extrémité distale des branches de pince (12, 13) près des mâchoires de blocage (22, 23), ouvertures par l'intermédiaire desquelles la pince peut être fixée sur le patient.

4. Dispositif selon l'une des revendications 1 à 3, **caractérisé en ce que** les extrémités allongées (18, 19) du ressort (16) sont appuyées sur deux boulons (20, 21) pouvant être enlevés des branches de pince (12, 13).

5. Dispositif selon la revendication 4, **caractérisé en ce que** les boulons (20, 21) sont logés dans la partie proximale des branches de pince (12, 13).

6. Dispositif selon l'une des revendications 1 à 5, **caractérisé en ce que** les branches de pince (12, 13) présentent deux paires de mâchoires de blocage, deux mâchoires de serrage (22, 23) et (32, 33) délimitant à chaque fois, dans la position de fermeture, une ouverture (24) et (34) à distance l'une de l'autre.

7. Dispositif selon la revendication 6, **caractérisé en ce que** la distance des ouvertures (24, 34) formées par les mâchoires de blocage (22, 23 et 32, 33) est réglable.

8. Dispositif selon l'une des revendications 1 à 7, **caractérisé en ce que** celui-ci est composé d'un matériau choisi dans le groupe des métaux, en particulier des métaux légers, et des matières plastiques.

9. Dispositif selon la revendication 8, **caractérisé en ce que** le métal est de l'aluminium.
